# DEMANDE DE BREVET EUROPEEN

(11) **EP 4 763 082 A1**
(43) Date de publication de la demande: **24.06.2026**
(21) Numéro de dépôt: 24307207.1
(22) Date de dépôt: 19.12.2024
(51) Int. Cl.: A61B 5/25, A61B 5/00, A61N 1/05, A61B 5/283, A61B 5/293, A61B 18/14

(54) **DISPOSITIF ENDOVASCULAIRE COMPORTANT UNE PLURALITE D'ELECTRODES**

(71) Demandeur: Centre Hospitalier Universitaire de Montpellier (CHUM), 34295 Montpellier Cedex 5 (FR)
(72) Inventeur: LEFEVRE, Pierre-Henri, 34980 SAINT CLEMENT DE RIVIERE (FR); DUIVON, Askan, 34090 MONTPELLIER (FR); LOUCHE, Hervé, 34980 SAINT GELY DU FESC (FR); JOURDAN, Franck, 34000 MONTPELLIER (FR)
(74) Mandataire: Santarelli

(57) **Abrégé**

La présente invention concerne un nouveau dispositif endovasculaire intégrant une pluralité d'électrodes indépendantes sur une hélice élastique permettant de positionner lesdits électrodes contre les parois des vaisseaux sanguins, et apte à passer dans une conformation étendue permettant le retrait du dispositif. En particulier, l'invention propose d'intégrer la pluralité d'électrodes sur une âme en matériau élastique de sorte à préserver au maximum l'élasticité de l'âme, et notamment en fixant la pluralité d'électrodes en surface de l'âme essentiellement par collage, et par les caractéristiques du tressage de la pluralité d'électrodes.

## Description

### DOMAINE TECHNIQUE DE L'INVENTION

La présente invention concerne le domaine des électrodes endovasculaires pour le recueil et/ou la stimulation des signaux électriques. En particulier, l'invention concerne un dispositif endovasculaire comportant une pluralité d'électrodes configurée sur une hélice super élastique apte à revenir dans une configuration étendue permettant le retrait dudit dispositif après implantation endovasculaire.

### DOMAINE TECHNIQUE DE L'INVENTION

Les électrodes endovasculaires sont des outils essentiels dans le domaine médical, conçus pour collecter des signaux électriques à l'intérieur des vaisseaux sanguins. Ces dispositifs sont particulièrement précieux pour la surveillance et l'enregistrement de l'activité électrique cardiaque ou intracrânienne. En outre, elles trouvent des applications potentielles dans le suivi des troubles neurologiques, la stimulation cérébrale profonde, et la recherche sur les maladies neurodégénératives. Au-delà des applications neurologiques, ces électrodes sont également utilisées pour surveiller l'activité cardiaque, évaluer la fonction musculaire, et dans le diagnostic de diverses pathologies vasculaires. Le développement de ces électrodes se concentre sur l'obtention de la biocompatibilité, de la flexibilité et de la précision pour fonctionner efficacement dans l'environnement complexe et électriquement bruyant du corps humain.

L'un des principaux défis dans ce domaine consiste en l'obtention d'une sensibilité et d'une précision élevées dans la collecte des signaux, ainsi que le maintien de la flexibilité nécessaire pour l'insertion et le positionnement dans les vaisseaux sanguins. De plus, les dispositifs doivent assurer une collecte de données cohérente malgré les défis posés par le flux sanguin et les mouvements naturels du corps. Des dispositifs tels que les stents sont conçus pour être fixés de manière sûre aux parois des vaisseaux sanguins. Cependant, l'ancrage des stents peut générer des problèmes tels que l'irritation des parois vasculaires, l'inflammation, ou même la formation de caillots sanguins. Ces complications soulignent l'importance de concevoir des dispositifs qui minimisent le contact excessif avec les parois des vaisseaux tout en assurant une fixation sûre et efficace.

Le document EP 4 282 463 A1 divulgue un appareil utilisé pour détecter ou stimuler l'activité du tissu nerveux. Cet appareil comprend au moins un dispositif intravasculaire qui est positionné à l'intérieur d'un vaisseau sanguin et équipé d'électrodes pour détecter ou stimuler l'activité du tissu nerveux à l'extérieur du vaisseau. Dans un mode de réalisation, le dispositif intravasculaire comporte un câble guide intégrant des électrodes et présentant une portion proximale linéaire et une portion distale hélicoïdale présentant un diamètre d'hélice apte à s'étendre ou se contracter. L'état de diamètre étendu permet de retenir l'hélice contre les parois du vaisseau. Une force expansive inférieure à celle d'un stent est apportée par un corps en spirale ayant une capacité de déformation, atteignant à la fois une petite force d'expansion et un effet de fixation de la position plus facilement qu'une endoprothèse rigide. Le dispositif est inséré au moyen d'un cathéter, et le câble assure un bon glissement du dispositif vis-à-vis du cathéter. Le document décrit l'utilisation d'un guide comportant une âme conductrice recouverte d'un isolant, avec des électrodes configurées en forme d'anneau autour de l'élément filaire et en contact électrique avec l'âme. L'appareil est conçu pour rester dans le vaisseau sanguin pendant de longues périodes, facilitant ainsi la surveillance ou la stimulation à long terme. Il n'est pas évoqué la possibilité de retrait du dispositif.

Le document AU2013205434 A1 porte également sur un dispositif intégrant un réseau d'électrodes intravasculaires flexibles pour la mesure et la stimulation corticales. Le dispositif présente deux positions : une position d'insertion synonyme d'un câble suffisamment mince pour se déplacer au travers des vaisseaux ; et une position déployée synonyme d'une hélice déployant les électrodes. Lorsque le câble guide est dans la position souhaitée, la position hélicoïdale déployée est induite thermiquement ou en enlevant un cathéter de recouvrement. Plusieurs modes de réalisation sont décrits, en général il est suggéré d'utiliser un câble guide flexible à mémoire de forme permettant d'apporter les deux positions au dispositif, et intégrant les électrodes. Les électrodes sont définies par des sections non isolées du câble guide, comportant optionnellement la fixation d'un matériau supplémentaire par brasage, soudage, dépôt chimique et d'autres méthodes de fixation au dispositif directement. Le câble guide peut être réalisé en nitinol, des alliages à mémoire de forme et des polymères pouvant être biodégradables/bio absorbables. Les électrodes peuvent être construits avec des câbles enroulant le câble guide ou disposés le long de ce dernier. Les électrodes sont réalisées en acier inox, en or, en platine etc. Ce dispositif est également conçu pour intégrer les électrodes sur une hélice élastique favorisant l'insertion du dispositif lorsque sa position étendue est retenue par un cathéter, toutefois, le document ne fait pas mention de la possibilité de revenir à cette position ni du retrait du dispositif.

Or, il serait souhaitable d'avoir un dispositif endovasculaire rétractable en toute sécurité du corps après son utilisation, minimisant ainsi les risques associés à une implantation permanente. En effet, la rétractabilité permettrait de réduire les complications potentielles telles que l'infection, l'inflammation chronique, et la formation de caillots sanguins. Dans le domaine du diagnostic médical, le caractère rétractable permettrait une évaluation précise et temporaire des conditions internes sans nécessiter d'implantation permanente. Cette caractéristique est particulièrement avantageuse pour des observations ponctuelles, comme le suivi de patients sous sédation en réanimation, ou pour surveiller l'évolution de pathologies transitoires à des moments critiques, notamment lors de leurs phases de déclenchement.

### EXPOSE DE L'INVENTION

La présente invention a comme objectif de fournir un nouveau dispositif endovasculaire rétractable intégrant une pluralité d'électrodes. A cette fin, l'invention propose un dispositif endovasculaire intégrant une pluralité d'électrodes disposée sur une portion en hélice d'une âme élastique. L'âme est configurée pour assurer une élasticité accrue de sa portion en hélice, lui permettant d'adopter une conformation sensiblement linéaire lors de son implantation dans un vaisseau, et de revenir dans cette conformation sensiblement linéaire lorsque ledit dispositif est tiré pour son retrait. C'est-à-dire que l'âme élastique est prévue pour que le diamètre d'un cylindre (en l'occurrence un cylindre fictif) formé par la portion en hélice, et la contenant, soit plus petit que le diamètre d'un vaisseau sanguin au moment de l'insertion et/ou de la rétractation du dispositif dans ce vaisseau sanguin.

En particulier, l'invention concerne un dispositif endovasculaire comportant une âme en matériau élastique comportant, à une extrémité terminale, une partie hélicoïdale intégrant une pluralité d'électrodes, et dans lequel chaque électrode de la pluralité d'électrodes est formée par un fil conducteur dédié comportant une partie conductrice formant une spirale serrée de fil embobiné autour de l'âme, et une partie de connexion destinée à relier ladite spirale avec une unité de traitement de signal à une extrémité proximale de l'âme, caractérisé en ce que
- les fils conducteurs des électrodes de ladite pluralité d'électrodes sont tressés en surface de l'âme, et en ce que :
   - ladite pluralité d'électrodes est fixée au niveau de ladite partie hélicoïdale au moyen du tressage des fils conducteurs sur l'âme, et par collage des spirales des électrodes avec l'âme.

La pluralité d'électrodes est ainsi intégrée sur l'âme de sorte à conserver et bénéficier au maximum des propriétés élastiques de la partie hélicoïdale.

De préférence, les spirales des électrodes sont collées sur l'âme au moyen des zones de colle appliquées au niveau des extrémités desdites spirales. Ces zones de colle permettent de garantir la stabilité mécanique et la continuité électrique de l'électrode sans besoin de soudure et/ou sertissage des extrémités des spirales.

Dans un mode de réalisation, les spirales des électrodes sont positionnées le long de l'âme au niveau de sa partie hélicoïdale, et dans ledit tressage, les parties de connexion des fils conducteurs sont enroulées selon une même direction autour de l'âme jusqu'à un niveau de positionnement de la spirale formée respectivement par chaque fil conducteur, et de sorte que la partie de connexion d'au moins une partie des fils conducteurs passe en-dessous d'une spirale ou des spirales des électrodes positionnés en amont et plus proches de l'extrémité proximale de l'âme, et de sorte que lesdits fils conducteurs sont maintenus sur l'âme au moyen desdites spirales. De préférence, les parties de connexion des fils conducteurs sont tressées enroulées de manière adjacente et avec un pas de spire espacé.

Le dispositif de l'invention peut également intégrer toute caractéristique ou combinaison de caractéristiques ci-après :
- les parties de connexion des fils conducteurs sont isolées électriquement ;
- l'âme est un fil de nitinol ;
- lesfils conducteurs sont en platine, en or, en argent, en acier inoxydable, en cuivre ou en alliage de CrCoMo, de préférence en platine.
- les électrodes sont isolées électriquement de l'âme ;
- les électrodes sont isolées de l'âme au moyen d'une face intérieure des électrodes face à l'âme, et lesdites électrodes sont formées par une spirale de fil comportant un revêtement isolant ayant été enlevé sur une surface externe de l'électrode destiné à être en contact du corps ;
- la colle est une colle photo polymérisable ;
- la pluralité d'électrodes est configurée pour fournir un signal indépendant pour chaque électrode ;
- la partie hélicoïdale intégrant la pluralité d'électrodes présente une élasticité apte à passer à une configuration étendue sensiblement linéaire ou de diamètre d'hélice réduit lors d'une mise en tension de ladite partie hélicoïdale à partir de l'extrémité proximale de l'âme, et sans besoin d'utilisation d'un élément de rétention de ladite configuration étendue ;
- l'âme intègre une deuxième pluralité d'électrodes destinée à recueillir des signaux extravasculaires, et étant situé d'un côté proximal de l'âme ; et
- l'âme comporte une partie linéaire reliant ladite partie hélicoïdale à l'extrémité proximale de l'âme.

D'autres caractéristiques et avantages de l'invention ressortiront du complément de description qui suit et qui se réfère aux figures annexées. Il va de soi que ce complément de description n'est donné qu'à titre d'illustration non limitatif de l'objet de l'invention.

### LISTE DE FIGURES

Figure 1. Une représentation schématique d'un dispositif endovasculaire intégrant une pluralité d'électrodes sur une âme comportant une partie hélicoïdale élastique selon l'invention.
Figure 2. Une représentation schématique du tressage de la pluralité d'électrodes sur l'âme du dispositif endovasculaire de l'invention selon un mode de réalisation.
Figure 3 Une représentation schématique d'une coupe transversale du dispositif endovasculaire au niveau d'une électrode de la pluralité d'électrodes selon un mode de réalisation intégrant 13 électrodes.

### DESCRIPTION DETAILLEE DE L'INVENTION

La présente invention a comme objectif de fournir un nouveau dispositif endovasculaire rétractable intégrant une pluralité d'électrodes pour la stimulation et/ou le recueil de signaux électriques. A cette fin, l'invention propose un dispositif endovasculaire intégrant la pluralité d'électrodes disposée sur une portion élastique en hélice du dispositif endovasculaire. En particulier, le dispositif est conçu pour assurer une super élasticité de la portion en hélice, lui permettant de revenir dans une conformation étendue sensiblement linéaire ou de diamètre réduit lorsqu'elle est soumise à une traction, facilitant ainsi son retrait. C'est-à-dire que le dispositif endovasculaire est prévu pour que le diamètre d'un cylindre (en l'occurrence un cylindre fictif) formé par cette portion en hélice, et la contenant, soit plus petit que le diamètre d'un vaisseau sanguin au moment de l'insertion et/ou de la rétractation du dispositif dans ce vaisseau sanguin. Il est un autre objectif de l'invention, que la pluralité d'électrodes soit conçue pour que chaque électrode fournisse un signal indépendant.

A ces fins, l'invention propose un dispositif 1 endovasculaire comportant une âme 2 présentant une première partie de forme linéaire, dite « partie proximale » d'un côté proximal P de l'âme, et une deuxième partie de forme hélicoïdale d'un côté terminal T (également appelé côté distal) de l'âme, dite « partie hélicoïdale » et sur laquelle une pluralité d'électrodes 30 est intégrée. La partie hélicoïdale présente une super élasticité lui permettant d'adopter plusieurs configurations, notamment une « configuration d'utilisation » dans laquelle la partie hélicoïdale conserve ladite forme hélicoïdale permettant le positionnement des électrodes contre les parois des vaisseaux 5 sanguins, et une configuration d'insertion ou de retrait du dispositif dans laquelle ladite hélice est étirée dans une « configuration étendue » sensiblement linéaire ou de diamètre d'hélice réduit.

On entend par « configuration étendue » que le diamètre du cylindre formé par la forme hélicoïdale est inférieur au diamètre du vaisseau sanguin dans laquelle est inséré le dispositif 1, ou duquel il est à retirer. Ce diamètre du cylindre est par la suite appelé « diamètre d'hélicoïde ou diamètre de la partie hélicoïdale ou encore diamètre d'hélice ». La configuration étendue présente donc un diamètre d'hélicoïde plus petit que lorsque le dispositif 1 est en place dans le vaisseau sanguin et que les électrodes touchent la paroi du vaisseau sanguin. Dans ce dernier cas, le diamètre d'hélicoïde correspond ou est sensiblement égal au diamètre du vaisseau sanguin. Ce dernier cas correspond à la configuration d'utilisation.

L'âme est réalisée dans un matériau élastique, de préférence super élastique, et suffisamment rigide pour maintenir les électrodes contre les parois des vaisseaux sanguins 5. De manière distinctive, la pluralité d'électrodes est tressée en surface de l'âme, ladite âme correspondant à un fil métallique auquel a été imparti la forme hélicoïdale sur la partie hélicoïdale. Dans un mode de réalisation préféré, cette âme est réalisée en fil de nitinol, un matériau reconnu pour son excellent rapport élasticité/rigidité et ses propriétés biocompatibles.

Optionnellement, l'âme est traitée préalablement avec un traitement de surface, tel qu'un électro-polissage. Par exemple, l'électro-polissage de l'âme en nitinol permet d'obtenir une surface particulièrement lisse et homogène, réduisant les micro-défauts susceptibles d'affecter la performance mécanique ou la biocompatibilité. Ce traitement favorise également la formation d'une couche protectrice d'oxyde de titane (TiO₂) à la surface du nitinol. Cette couche, générée par passivation naturelle après électro-polissage, agit comme une barrière efficace pour limiter le relargage de nickel contenu dans l'alliage, améliorant ainsi la biocompatibilité du dispositif.

L'invention propose également d'intégrer la pluralité d'électrodes sur l'âme 2 de sorte à conserver au maximum les propriétés de super élasticité de l'âme. En particulier, le dispositif 1 comporte un tressage de la pluralité d'électrodes 30 en surface de l'âme 2 (i.e., le tressage correspond à un enroulement des fils correspondant chacun à l'une des électrodes sur l'âme), et cette pluralité d'électrodes 30 est fixée essentiellement par les caractéristiques du tressage (i.e., par les caractéristiques de l'enroulement même des fils autour de l'âme) de la pluralité d'électrodes et par collage des électrodes 30 sur l'âme 2.

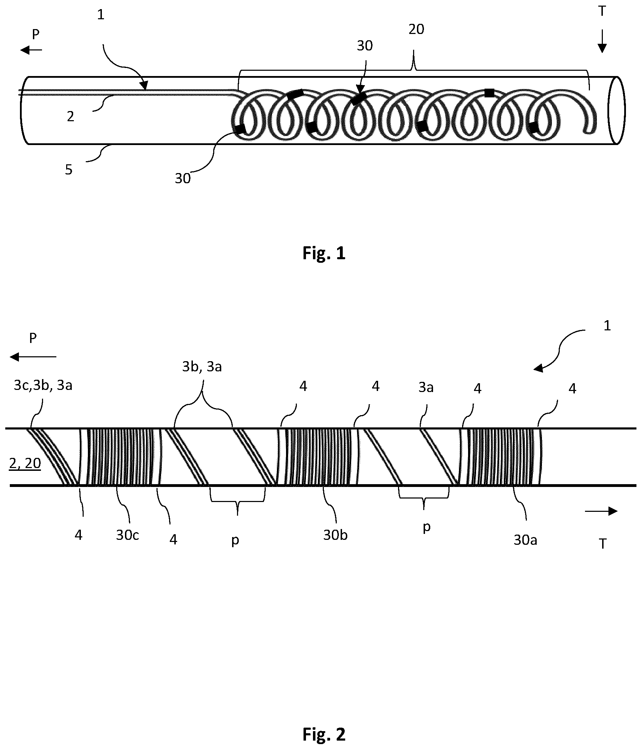

La figure 2 illustre les particularités du tressage de la pluralité d'électrodes 30 selon un mode de réalisation préféré sur la partie hélicoïdale 20. Pour faciliter la compréhension du tressage, la forme hélicoïdale de l'âme est ici omise. La pluralité d'électrodes comporte plusieurs électrodes 30a, 30b, 30c, chaque électrode étant formée par une spirale serrée d'un fil conducteur 3a, 3b, 3c, enroulé autour de l'âme 2. Par « spirale serrée » on entend que les spires de l'enroulement sont jointives pour chaque électrode, et que le fil conducteur est directement contre l'âme 2. Les fils conducteurs peuvent être en or, argent, cuivre, acier inox, un alliage tel que le CrCoMo ou en platine. Dans un mode de réalisation préféré, les fils conducteurs 3a, 3b, 3c, sont des fils en platine pour conserver l'élasticité de l'hélice, et permettant une bonne transmission de signal avec une faible section de fil, et donc, permettant l'intégration d'un nombre important électrodes, typiquement plus de 10 électrodes, voire plus de 15 ou encore plus de 20 électrodes.

De manière particulière, les électrodes 30a, 30b, 30c sont fixées à l'âme 2 en appliquant des zones de colle 4 au niveau des extrémités de chacune des spirales définissant lesdites électrodes 30a, 30b, 30c. Ces zones de colle 4 permettent de garantir la stabilité mécanique et la continuité électrique de l'électrode sans besoin de soudure et/ou sertissage des extrémités des spirales. Cette méthode de fixation est donc très avantageuse en ce qu'elle permet de conserver au maximum les propriétés élastiques de l'âme. Cette colle est par exemple une colle photo-polymérisable biocompatible, tel qu'une colle photopolymérisant UV de type acrylate.

Au moins au niveau de la partie hélicoïdale, ces zones de colle 4 seront, de préférence, le principal moyen de fixation supplémentaire utilisé pour fixer les électrodes 30. La pluralité d'électrodes est également maintenue sur l'âme grâce aux caractéristiques du tressage, ledit tressage comportant optionnellement un revêtement final appliqué hors des spirales d'électrodes. En outre, le fil conducteur 3 de chaque électrode 30a, 30b, 30c comprend une partie conductrice, laquelle forme l'électrode afférente, et une partie de connexion. La partie de connexion s'étend enroulée le long de l'âme 2 depuis la partie conductrice (i.e., l'électrode) jusqu'à la partie proximale, voire jusqu'à l'extrémité proximale de la partie proximale. La partie conductrice est, de préférence, non isolée électriquement au niveau de sa surface qui est destinée à être en contact avec la paroi du vaisseau sanguin lorsque le dispositif 1 est dans ledit vaisseau sanguin. C'est-à-dire que la portion de chaque électrode qui est en contact avec la paroi du vaisseau sanguin n'est pas isolée électriquement. Cette portion non isolée est appelée la « surface active » de l'électrode. De préférence également, la partie de connexion est isolée électriquement.

La partie de connexion peut être telle qu'elle passe, le cas échéant, en-dessous d'une spirale d'une ou des électrodes positionnées en amont dans ledit tressage de la pluralité d'électrodes, c'est-à-dire plus proches de l'extrémité proximale de l'âme 2. Ce tressage permet d'utiliser les spirales des électrodes 30 pour sécuriser les parties de connexion de fils conducteurs 3 formant chaque électrode. Comme illustré sur la figure 2, chaque électrode 30a, 30b, 30c est formée par un fil indépendant fournissant un signal indépendant à une unité de traitement et d'acquisition de signal. De même, la conception des électrodes sous forme de spirale en utilisant un fil conducteur dédié permet de mieux se conformer au design hélicoïdal, et d'éviter également les soudures avec des fils de connexion pour la transmission du signal, de sorte que l'élasticité de l'âme est préservée.

Autrement dit, la partie de connexion d'un fil conducteur 3 peut être placée le long de l'âme, typiquement par enroulement autour de l'âme, de sorte que cette partie de connexion soit maintenue sur l'âme par la partie conductrice formant une autre électrode de la pluralité d'électrode. C'est-à-dire que la partie de connexion d'au moins une partie des fils conducteurs formant la pluralité d'électrodes circule le long de l'âme de sorte à passer sous les électrodes qui sont situées en amont sur la partie hélicoïdale. On entend par « électrode située en amont » une électrode située entre la partie proximale et l'électrode afférente au fil conducteur considéré (passant sous l'électrode située en amont). Cette électrode située en amont est donc située en amont par rapport à l'électrode afférente au fil considéré.

En particulier, chaque électrode de la pluralité d'électrodes est positionnée à une distance donnée de la partie proximale, typiquement de l'extrémité proximale de la partie proximale. La pluralité d'électrodes comprend ainsi une première électrode positionnée sur la partie hélicoïdale telle que sa distance à la partie proximale est la plus petite, comparativement à la distance des autres électrodes de la pluralité d'électrodes à la partie proximale (typiquement à l'extrémité proximale de la partie proximale). Chaque électrode, autre que la première électrode, a son fil conducteur afférent qui est positionné sur l'âme de sorte qu'il circule, ou passe, sous les électrodes de la pluralité d'électrodes pour lesquelles la distance à la partie proximale est plus petite que l'électrode en question (incluant donc également la première électrode).

A titre d'illustration, en lien avec la figure 2, le fil conducteur afférent à l'électrode 30a passe sous l'électrode 30b et sous l'électrode 30c, lesquelles se trouvent en amont de l'électrode 30a. Le fil conducteur afférent à l'électrode 30b passe sous l'électrode 30c, en amont de l'électrode 30b. L'électrode 30c correspond ici donc à la première électrode mentionnée ci-avant.

Ainsi, au niveau d'une électrode donnée, les fils conducteurs afférents aux électrodes situées en aval (i.e., afférents à chacune des électrodes dont la distance à la partie proximale est plus grande que la distance de l'électrode donnée à la partie proximale) sont positionnés sous l'électrode donnée.

Les parties de connexion des différents fils conducteurs sont, par exemple, enroulées sur l'âme, adjacentes les unes des autres selon une même direction, évitant ainsi les croisements de fils pour maintenir la flexibilité et l'intégrité mécanique du dispositif. La figure 2 illustre un mode de réalisation non limitatif avec trois électrodes 30a, 30b, 30c. On observe, d'un côté proximal P de la partie hélicoïdale, les fils 3c, 3b, 3a enroulés adjacents autour de l'âme 2, notamment leur partie de connexion, avec un pas p de spire espacé. Chaque fil conducteur s'enroule selon le pas p de spire jusqu'à sa partie conductrice. Chaque électrode (i.e., chaque partie conductrice) est formée par un bobinage serré du fil conducteur afférent. Les fils 3b et 3a sont sécurisés à l'âme par la spirale correspondant à l'électrode 30c, et traversent par le dessous la spirale de l'électrode 30c pour continuer le tressage selon le même principe en direction du côté terminal T de la partie hélicoïdale 20. Le pas de spire espacé, dans l'enroulement des parties de connexion, permet de diminuer la longueur totale des fils conducteurs, et donc de limiter l'impédance et avoir une bonne qualité de réception des signaux électriques. D'autre part, le côté jointif entre les parties de connexion (i.e. adjacent) permet de mieux maintenir les extrémités des spirales et donc d'assurer la continuité électrique des spirales, c'est-à-dire les plots des électrodes. Par pas de spire espacé on entend dans la présente invention qu'une distance minimale, ici le pas p, est maintenue entre chaque tour d'un ou plusieurs fils conducteurs enroulés le long de l'âme 2.

Les fils conducteurs 3 sont isolés électriquement de l'âme afin d'éviter des courts circuits avec le matériau métallique conducteur de l'âme 2. A cette fin, les fils conducteurs comportent un revêtement isolant biocompatible couvrant entièrement les parties de connexion des fils, et la face intérieure des spirales en contact avec l'âme. Dans un mode de réalisation, le tressage de la pluralité d'électrodes est fait avec des fils conducteurs comportant un revêtement isolant, et ce revêtement est ensuite enlevé au niveau de la surface extérieure des spirales. La face extérieure des spirales correspond à la surface active des électrodes, i.e., leur surface destinée à être en contact du corps (typiquement la paroi d'un vaisseau sanguin, telle une artère) et à recueillir le signal, et leur face intérieure correspond à leur surface en contact de l'âme 2.

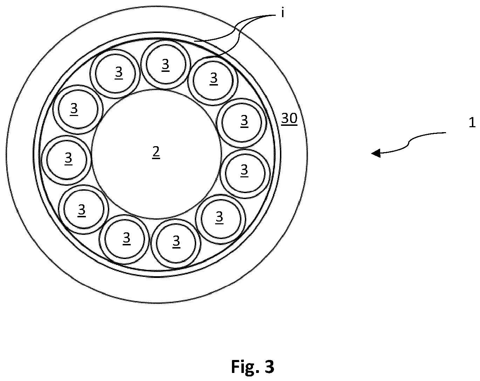

La figure 3 montre une représentation schématique d'une coupe transversale au niveau d'une spirale d'électrode proximale de l'hélice, en-dessous de laquelle passent les parties de connexion des fils conducteurs des électrodes positionnées en aval. La surface externe de l'âme 2 est recouverte par les parties de connexion des fils conducteurs des électrodes plus distales du dispositif. Il est également illustré les zones de revêtement isolant i sur les fils conducteurs 3 au niveau des parties de connexion et au niveau de la face intérieure de la spirale d'électrode 30. Le revêtement isolant est par exemple un revêtement en polyamide. La section totale d'un câble ainsi formé est d'environ 70-650µm, et dans lequel la section de fil en nitinol est d'environ 50-450 µm, et la section de fils conducteurs de 10-100 µm.

Le nombre d'électrodes de la pluralité d'électrodes pourra être facilement augmenté en augmentant la longueur de l'hélice, et permettre lorsque souhaité, un pas de spire p espacé pour l'enroulement des parties de connexion autour de l'âme 2. Le nombre de tours de spire des électrodes détermine la surface active, et dans un mode de réalisation non limitatif, la taille de l'électrode (notamment leur longueur selon une direction orthogonale la direction d'enroulement du fil conducteur afférent, c'est-à-dire leur longueur selon la direction d'élongation de l'âme) sera d'environ 1-2 mm. Le diamètre de la partie hélicoïdale sera choisi en fonction du vaisseau sanguin dans lequel est destiné à être implanté le dispositif, et sera de maximum environ 10 mm, la taille maximale d'un vaisseau. Un tour de spire de la partie hélicoïdale, ainsi que l'espacement entre les électrodes seront choisis en fonction de l'utilisation souhaitée pour le dispositif. De même, dans un mode de réalisation le dispositif intègre des électrodes tressées de manière équivalente sur le côté proximal P de l'âme, tel que sur l'extrémité proximale linéaire de l'âme destiné à recueillir le signal sur l'extérieur du patient. Dans un mode de réalisation, le dispositif est fabriqué en apportant dans une première étape la forme hélicoïdale à l'extrémité terminale de l'âme ayant de préférence suivi un pré-traitement de surface. Dans une deuxième étape, la pluralité d'électrodes est tressée en surface de l'âme. Enfin, un revêtement final est de préférence appliqué sur le tressage de la pluralité d'électrodes, notamment hors des zones des spirales des électrodes, pour améliorer la lubrification et la glisse du dispositif lors des manipulations intravasculaires d'insertion ou de retrait du dispositif, et/ou pour améliorer la biocompatibilité. Naturellement, ce revêtement permet d'améliorer également le maintien de la pluralité d'électrodes sur l'âme. Par exemple, ce revêtement final est un revêtement mince hydrophile tel qu'un revêtement en polytétrafluoroéthylène (PFTE) ou en hydrogel hydrophile

Dans un mode de réalisation, le dispositif endovasculaire de l'invention est un microguide neurovasculaire pour l'enregistrement de l'activité électrique cérébrale, et trouve une application dans une méthode de recueil de signaux intracrâniens pour effectuer un bilan pré-chirurgical des épilepsies pharmaco résistantes.

## Revendications

1. Dispositif (1) endovasculaire comportant une âme (2) en matériau élastique comportant, à une extrémité terminale, une partie hélicoïdale intégrant une pluralité d'électrodes (30), et dans lequel chaque électrode (30a, 30b, 30c) de la pluralité d'électrodes (30) est formée par un fil conducteur (3a, 3b, 3c) dédié comportant une partie conductrice formant une spirale serrée de fil embobiné autour de l'âme (2), et une partie de connexion destinée à relier ladite spirale avec une unité de traitement de signal à une extrémité proximale de l'âme, **caractérisé en ce que**
- les fils conducteurs (3a, 3b, 3c) des électrodes ((30a, 30b, 30c) de la pluralité d'électrodes (30) sont tressés en surface de l'âme (2), et **en ce que** :
- la pluralité d'électrodes (30) est fixée au niveau de ladite partie hélicoïdale au moyen du tressage des fils conducteurs sur l'âme (2), et par collage des spirales des électrodes avec l'âme (2).

2. Dispositif selon la revendication 1, dans lequel les spirales des électrodes (30a, 30b, 30c) sont collées sur l'âme (2) au moyen des zones de colle (4) appliquées au niveau des extrémités desdites spirales.

3. Dispositif selon l'une des revendications précédentes, dans lequel les spirales des électrodes (30a, 30b, 30c) sont positionnées le long de l'âme (2) au niveau de sa partie hélicoïdale, et dans ledit tressage, les parties de connexion des fils conducteurs sont enroulées selon une même direction autour de l'âme (2) jusqu'à un niveau de positionnement de la spirale formée respectivement par chaque fil conducteur (3a, 3b, 3c), et de sorte que la partie de connexion d'au moins une partie des fils conducteurs passe en-dessous d'une spirale ou des spirales des électrodes positionnés en amont (30b, 30c) et plus proches de l'extrémité proximale de l'âme (2), et de sorte que lesdits fils conducteurs sont maintenus sur l'âme (2) au moyen desdites spirales.

4. Dispositif selon la revendication 3, dans lequel les parties de connexion des fils conducteurs sont tressées enroulées de manière adjacente et avec un pas de spire (p) espacé.

5. Dispositif selon l'une des revendications précédentes, dans lequel les parties de connexion des fils conducteurs sont isolées électriquement.

6. Dispositif selon l'une des revendications précédentes, dans lequel l'âme est un fil de nitinol.

7. Dispositif selon l'une des revendications précédentes, dans lequel les fils conducteurs (3a, 3b, 3c) sont en platine, en or, en argent, en acier inoxydable, en cuivre ou en alliage de CrCoMo, de préférence en platine.

8. Dispositif selon l'une des revendications précédentes, dans lequel les électrodes (30a, 30b, 30c) sont isolées électriquement de l'âme (2).

9. Dispositif selon l'une des revendications précédentes, dans lequel les électrodes sont isolées de l'âme (2) au moyen d'une face intérieure des électrodes face à l'âme (2), et lesdites électrodes (30a, 30b, 30c) sont formées par une spirale de fil comportant un revêtement isolant ayant été enlevé sur une surface externe des électrodes destinée à être en contact du corps.

10. Dispositif selon l'une des revendications précédentes, dans lequel la colle est une colle photo polymérisable.

11. Dispositif selon l'une des revendications précédentes, dans lequel la pluralité d'électrodes est configurée pour fournir un signal indépendant pour chaque électrode.

12. Dispositif selon l'une des revendications précédentes, dans lequel la partie hélicoïdale intégrant la pluralité d'électrodes (30) présente une élasticité apte à passer à une configuration étendue sensiblement linéaire ou de diamètre d'hélice réduit lors d'une mise en tension de ladite partie hélicoïdale à partir de l'extrémité proximale de l'âme (2), et sans besoin d'utilisation d'un élément de rétention de ladite configuration étendue.

13. Dispositif selon l'une des revendications précédentes, dans lequel l'âme intègre une deuxième pluralité d'électrodes destinée à recueillir des signaux extravasculaires, et étant situé d'un côté proximal de l'âme (2).

14. Dispositif selon l'une des revendications précédentes, dans lequel l'âme (2) comporte une partie linéaire reliant ladite partie hélicoïdale à l'extrémité proximale de l'âme.
